(19) Europäisches Patentamt
European Patent Office
Office européen des brevets

(11) EP 0 722 713 B1

(12) **FASCICULE DE BREVET EUROPEEN**

(45) Date de publication et mention de la délivrance du brevet:
**06.08.1997 Bulletin 1997/32**

(51) Int. Cl.$^6$: **A61K 7/13**

(21) Numéro de dépôt: **96400115.0**

(22) Date de dépôt: **17.01.1996**

(54) **Composition de teinture d'oxydation des fibres kératiniques et procédé de teinture mettant en oeuvre cette composition**

Oxydationsfärbemittel für keratinische Fasern und Färbeverfahren mit demselben Mittel

Composition for oxidative dyeing of keratin fibers and process of dyeing using that composition

(84) Etats contractants désignés:
**AT BE CH DE DK ES FR GB GR IE IT LI LU MC NL PT SE**

(30) Priorité: **19.01.1995 FR 9500587**

(43) Date de publication de la demande:
**24.07.1996 Bulletin 1996/30**

(73) Titulaire: **L'OREAL**
**75008 Paris (FR)**

(72) Inventeurs:
- **Bone, Eric**
  **F-92110 Clichy (FR)**
- **de la Mettrie, Roland**
  **F-78110 Le Vesinet (FR)**

(74) Mandataire: **Andral, Christophe André Louis**
**L'OREAL**
**Centre de Recherche Charles Zviak**
**Département Propriété Industrielle**
**90, rue du Général Roguet**
**92583 Clichy Cedex (FR)**

(56) Documents cités:
**WO-A-93/10744**
**FR-A- 2 362 116**
**GB-A- 2 054 665**
**GB-A- 2 180 215**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 722 713 B1

Printed by Rank Xerox (UK) Business Services
2.14.12/3.4

## Description

L'invention a pour objet une composition pour la teinture d'oxydation des fibres kératiniques, en particulier des fibres kératiniques humaines telles que les cheveux, comprenant de la paraphénylènediamine et/ou de la 2-méthyl paraphénylènediamine, en association avec au moins un dérivé de métaphénylènediamine convenablement sélectionné et au moins un dérivé de paraphénylènediamine tertiaire convenablement sélectionné, ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).
Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans le brevet français FR-2 362 116, des compositions pour la teinture d'oxydation des fibres kératiniques renfermant au moins un dérivé de métaphénylènediamine à titre de coupleur, en association avec au moins un précurseur de colorant d'oxydation. Les colorations obtenues avec ces compositions ne sont toutefois pas entièrement satisfaisantes, notamment en ce qui concerne la tenue de ces colorations vis à vis des diverses agressions que peuvent subir les cheveux et en particulier les shampooings et la lumière.

La présente invention vise à proposer de nouvelles compositions pour la coloration d'oxydation des fibres kératiniques et en particulier des fibres kératiniques humaines telles que les cheveux qui présentent de très bonnes propriétés tinctoriales.

Ainsi, la demanderesse vient maintenant de découvrir qu'il est possible d'obtenir de nouvelles teintures particulièrement résistantes, qui engendrent des colorations intenses et peu sélectives, en associant :

- de la paraphénylènediamine et/ou de la 2-méthyl paraphénylènediamine et/ou l'un de leurs sels d'addition avec un acide,
- au moins un dérivé de métaphénylènediamine spécifique de formule (I) définie ci-après,
- et au moins un dérivé de paraphénylènediamine tertiaire spécifique de formule (II) définie ci-après.

Cette découverte est à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture :

- au moins une première base d'oxydation choisie parmi la paraphénylènediamine, la 2-méthyl paraphénylènediamine et leurs sels d'addition avec un acide,
- au moins un coupleur choisi parmi les dérivés de métaphénylènediamine de formule (I) suivante :

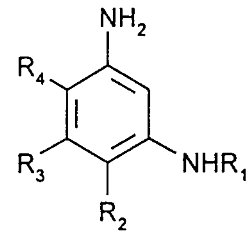

(I)

dans laquelle :

$R_1$ représente un atome d'hydrogène, un radical alkyle en $C_1$-$C_4$, monohydroxyalkyle en $C_1$-$C_4$ ou polyhydroxyalkyle en $C_2$-$C_4$ ;
$R_2$ et $R_3$, identiques ou différents, représentent un atome d'hydrogène, un radical alkyle en $C_1$-$C_4$ ou hydroxyalkoxy en $C_1$-$C_4$ ;
$R_4$ représente un radical alkoxy en $C_1$-$C_4$, aminoalkoxy en $C_1$-$C_4$, monohydroxyalkoxy en $C_1$-$C_4$, polyhydroxyalkoxy en $C_2$-$C_4$ ou un radical 2,4-diaminophénoxyalkoxy, sous réserve que lorsque $R_1$ désigne un atome d'hydrogène, alors $R_2$ et $R_4$ ne désignent pas simultanément un radical β-hydroxyéthyloxy, et que lorsque $R_1$, $R_2$ et $R_3$ désignent simultanément un atome d'hydrogène, alors $R_4$ est différent de méthoxy,
et leurs sels sels d'addition avec un acide,

- et au moins une deuxième base d'oxydation choisie parmi les dérivés de paraphénylènediamine tertiaire de formule (II) suivante :

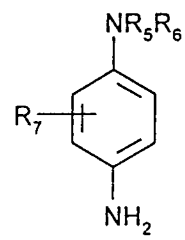

(II)

dans laquelle :

$R_5$ représente un radical alkyle en $C_1$-$C_4$, monohydroxyalkyle en $C_1$-$C_4$ ou polyhydroxyalkyle en $C_2$-$C_4$;
$R_6$ représente un radical monohydroxyalkyle en $C_1$-$C_4$ ou polyhydroxyalkyle en $C_2$-$C_4$ ;
$R_7$ représente un atome d'hydrogène, un radical alkyle en $C_1$-$C_4$, alkoxy en $C_1$-$C_4$ ou un atome d'halogène,
et leurs sels d'addition avec acide.

Les colorations obtenues avec les compositions ci-dessus présentent une bonne puissance tinctoriale et d'excellentes propriétés de résistance à la fois vis à vis des agents atmosphériques tels que la lumière et les intempéries et vis à vis de la transpiration et des différents traitements que peuvent subir les cheveux (shampooings, déformations permanentes). Ces propriétés sont particulièrement remarquables, notamment en ce qui concerne la résistance des colorations obtenues vis à vis des shampooings et de la lumière.

L'invention a également pour objet un procédé de teinture d'oxydation des fibres kératiniques mettant en oeuvre cette composition.

Les sels d'addition avec un acide utilisables dans le cadre des compositions tinctoriales de l'invention peuvent être notamment choisis parmi les chlorhydrates, les bromhydrates, les sulfates et les tartrates.

Parmi les dérivés de métaphénylénediamine formule (I) ci-dessus, on peut plus particulièrement citer le 3,5-diamino 1-éthyl 2-méthoxybenzène, le 3,5-diamino 2-méthoxy 1-méthyl benzène, le 2,4-diamino 1-éthoxybenzène, le 1,3-bis-(2,4-diaminophénoxy) propane, le bis-(2,4-diaminophénoxy) méthane, le 1-(β-aminoéthyloxy) 2,4-diamino benzène, le 2-amino 1-(β-hydroxyéthyloxy) 4-méthylamino benzène, le 2,4-diamino 1-éthoxy 5-méthyl benzène, le 2,4-diamino 5-(β-hydroxyéthyloxy) 1-méthylbenzène, le 2,4-diamino 1-(β,γ-dihydroxypropyloxy) benzène, le 2,4-diamino 1-(β-hydroxyéthyloxy) benzène, le 2-amino 4-N-(β-hydroxyéthyl) amino 1-méthoxy benzène, et leurs sels d'addition avec un acide.

Parmi les dérivés de paraphénylènediamine tertiaire de formule (II) ci-dessus, on peut plus particulièrement citer le 1-(N-éthyl, N'-β-hydroxyéthyl)amino 4-amino benzène, le 1-N,N'-bis-(β-hydroxyéthyl)amino 4-amino benzène, le 1-N,N'-bis-(β,γ-dihydroxypropyl)amino 4-amino benzène, et leurs sels d'addition avec un acide.

La paraphénylènediamine et/ou la 2-méthyl paraphénylènediamine et/ou le ou les sels d'addition de ces composés avec un acide représentent de préférence de 0,0005 à 10 % en poids environ du poids total de la composition tinctoriale, et encore plus préférentiellement de 0,05 à 7 % en poids environ.

Le ou les dérivés de métaphénylènediamine formule (I) conformes à l'invention représentent de préférence de 0,0001 à 5 % en poids environ du poids total de la composition tinctoriale, et encore plus préférentiellement de 0,005 à 3 % en poids environ.

Le ou les dérivés de paraphénylènediamine tertiaire de formule (II) conformes à l'invention représentent de préférence de 0,0005 à 10 % en poids environ du poids total de la composition tinctoriale et encore plus préférentiellement de 0,05 à 7 % en poids environ.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; le glycérol ; les glycols et éthers de glycols comme le 2-butoxyéthanol, le propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale telle que définie précédemment est généralement compris entre 2 et 12. Il peut être ajusté à la valeur désirée au moyen d'agents acidifiants ou alcalinisants habituellement utilisés en teinture des fibres kératiniques.

Parmi les agents acidifiants, on peut citer, à titre d'exemples, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants, on peut citer, à titre d'exemples, l'ammoniaque, les carbonates alcalins, les alcanolamines telles que les mono-, di- et triéthanolamines ainsi que leurs dérivés, les hydroxydes de sodium ou de potassium et les composés de formule (III) suivante :

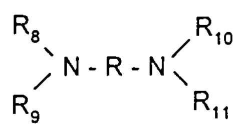

(III)

dans laquelle R est un reste propylène éventuellement substitué par un groupement hydroxyle ou un radical alkyle en $C_1$-$C_4$ ; $R_8$, $R_9$, $R_{10}$ et $R_{11}$, identiques ou différents, représentent un atome d'hydrogène, un radical alkyle en $C_1$-$C_4$ ou hydroxyalkyle en $C_1$-$C_4$.

La composition tinctoriale conforme à l'invention peut encore contenir, en plus des colorants définis ci-dessus, d'autres bases d'oxydation et/ou d'autres coupleurs et/ou des colorants directs, notamment pour modifier les nuances ou les enrichir en reflets.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres à colorer la composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou

séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 40 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 2 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

**EXEMPLES**

**EXEMPLES 1 à 3**

On a préparé les compositions tinctoriales conformes à l'invention, suivantes (teneurs en grammes) :

| EXEMPLE | 1 | 2 | 3 |
|---|---|---|---|
| Paraphénylènediamine | 0,7 | | 0,2 |
| 2-méthyl paraphénylènediamine | | 0,2 | |
| Dichlorhydrate de 2,4-diamino 1-(β-hydroxyéthyloxy) benzène | 0,08 | 0,05 | |
| 2-amino 4-N-(β-hydroxyéthyl) amino 1-méthoxy benzène | | | 0,1 |
| Sulfate de 1-N,N'-bis-(β-hydroxyéthyl) amino 4-amino benzène | 0,15 | 0,3 | 0,1 |
| Résorcine | 0,5 | 0,4 | 0,35 |
| Support de teinture commun (*) | (*) | (*) | (*) |
| Eau q.s.p. | 100 g | 100 g | 100 g |

(*) <u>support de teinture commun</u> :

| | | |
|---|---|---|
| - Alcool oléique polyglycérolé à 2 moles de glycérol | 4,0 | g |
| - Alcool oléique polyglycérolé à 4 moles de glycérol, à 78 % de matières actives (M.A.) | 5,69 | g M.A. |
| - Acide oléique | 3,0 | g |
| - Amine oléique à 2 moles d'oxyde d'éthylène vendue sous la dénomination commerciale ETHOMEEN O12 par la société AKZO | 7,0 | g |
| - Laurylamino succinamate de diéthylaminopropyle, sel de sodium à 55 % de M.A. | 3,0 | g M.A. |
| - Alcool oléique | 5,0 | g |

| | | |
|---|---|---|
| - Diéthanolamide d'acide oléique | 12,0 | g |
| - Propylèneglycol | 3,5 | g |
| - Alcool éthylique | 7,0 | g |
| - Dipropylèneglycol | 0,5 | g |
| - Monométhyléther de propylèneglycol | 9,0 | g |
| - Métabisulfite de sodium en solution aqueuse, à 35 % de M.A. | 0,455 | g M.A. |
| - Acétate d'ammonium | 0,8 | g |
| - Antioxydant, séquestrant | q.s. | |
| - Parfum, conservateur | q.s. | |
| - Ammoniaque à 20 % de $NH_3$ | 10,2 | g |

Au moment de l'emploi, on a mélangé chaque composition tinctoriale avec une quantité égale d'une composition oxydante constituée par une solution d'eau oxygénée à 20 volumes (6 % en poids) et présentant un pH d'environ 3.

Chaque mélange obtenu présentait un pH d'environ 10,2 et a été appliqué pendant 30 minutes sur des mèches de cheveux gris naturels à 90 % de blancs. Les mèches de cheveux ont ensuite été rincées, lavées avec un shampooing standard puis séchées.

Les mèches de cheveux ont été teintes dans les nuances figurant dans le tableau ci-dessous :

| EXEMPLE | NUANCE SUR CHEVEUX NATURELS |
|---|---|
| 1 | châtain cendré |
| 2 | blond foncé |
| 3 | blond cendré |

**EXEMPLES 4 à 6 COMPARATIFS**

On a préparé les compositions tinctoriales suivantes :

| EXEMPLE | 4(**) | 5(**) | 6(***) |
|---|---|---|---|
| | | | |
| Paraphénylènediamine (en mole) | $3.10^{-3}$ | | $2,5.10^{-3}$ |
| | | | |
| Dichlorhydrate de 2,4-diamino 1-(β-hydroxyéthyloxy) benzène (en mole) | $3.10^{-3}$ | $3.10^{-3}$ | $0,5.10^{-3}$ |
| | | | |
| Sulfate de 1-N,N'-bis-(β-hydroxyéthyl) amino 4-amino benzène (en mole) | | $3.10^{-3}$ | $3.10^{-3}$ |
| | | | |
| Support de teinture commun (*) | (*) | (*) | (*) |
| Eau q.s.p. | 100 g | 100 g | 100 g |

(*) : voir ci-avant
(**) : exemples ne faisant pas partie de l'invention
(***) : exemple faisant partie de l'invention

Chaque composition tinctoriale comprend, au total, $6.10^{-3}$ mole de colorants.

Au moment de l'emploi, on a mélangé chaque composition tinctoriale avec une quantité égale d'une composition oxydante constituée par une solution d'eau oxygénée à 20 volumes (6 % en poids) et présentant un pH d'environ 3.

Chaque mélange obtenu présentait un pH d'environ 10,2 et a été appliqué pendant 30 minutes sur des mèches de cheveux gris permanentés à 90 % de blancs. Les mèches de cheveux ont ensuite été rincées, lavées avec un shampooing standard puis séchées.

La couleur des mèches a ensuite été évaluée dans le système MUNSELL au moyen d'un colorimètre CM 2002 MINOLTA.

Selon la notation MUNSELL, une couleur est définie par l'expression **H V/C** dans laquelle les trois paramètres désignent respectivement la teinte ou Hue (**H**), l'intensité ou Value (**V**) et la pureté ou Chromaticité (**C**), la barre oblique de cette expression est simplement une convention et n'indique pas un ratio.

Les mèches de cheveux ainsi teintes ont ensuite été soumises à un test de résistance aux lavages(machine Ahiba-Texomat).

Pour ce faire, les mèches de cheveux ont été placées dans un panier que l'on a immergé dans une solution d'un shampooing standard. Le panier a été soumis à un mouvement de va-et-vient vertical de fréquence variable ainsi qu'à un mouvement de rotation qui reproduisent l'action d'un frottement manuel, ce qui engendre la formation de mousse.

Après 3 minutes d'épreuve, on a retiré les mèches que l'on a rincées puis séchées. Les mèches teintes ont été soumises à 8 épreuves de shampooing consécutives.

La couleur des mèches a été ensuite évaluée à nouveau dans le système MUNSELL au moyen d'un colorimètre CM 2002 MINOLTA.

La différence entre la couleur de la mèche avant les shampooings et la couleur de la mèche après les shampooings a été calculée en appliquant la formule de NICKERSON:

$\Delta E = 0,4\ Co\Delta H + 6\Delta V + 3\ \Delta C$ , telle que décrite par exemple dans "Couleur, Industrie et Technique" ; pages 14-17 ; vol. n° 5 ; 1978.

Dans cette formule, ΔE représente la différence de couleur entre deux mèches, ΔH, ΔV et ΔC représentent la variation en valeur absolue des paramètres H, V et C et Co représente la pureté de la mèche par rapport à laquelle on désire évaluer la différence de couleur.

Les résultats sont donnés dans le tableau ci-dessous :

| EXEMPLE | Couleur des cheveux avant les shampooings | Couleur des cheveux après les shampooings | Dégradation de la couleur | | | |
|---|---|---|---|---|---|---|
| | | | ΔH | ΔV | ΔC | **ΔE** |
| | | | | | | |
| **4** | 8,55 PB 0,3 / 1,7 | 9,5 PB 1,9 / 2,1 | 0,95 | 1,6 | 0,4 | **11,5** |
| **5** | 6,75 PB 0,3 / 3,4 | 1,25 PB 1,6 / 4,1 | 5,5 | 1,3 | 0,7 | **17,4** |
| **6** | 7,95 PB 0,3 / 1,6 | 7,0 PB 0,9 / 2,8 | 0,95 | 0,6 | 1,2 | **7,8** |

Ces résultats montrent que la composition de l'exemple 6 conforme à l'invention, c'est à dire comprenant de la paraphénylènediamine, un dérivé de métaphénylènediamine de formule (I) conforme à l'invention et un dérivé de paraphénylènediamine tertiaire de formule (II) conforme à l'invention, conduit à une coloration résistant beaucoup mieux aux shampooings que les colorations obtenues avec les compositions des exemples 4 et 5 ne faisant pas partie de l'invention car elles ne contiennent, chacune, que deux seulement des trois composés ci-dessus.

**Revendications**

**1.** Composition pour la teinture d'oxydation des fibres kératiniques et en particulier des fibres kératiniques humaines telles que les cheveux, caractérisée par le fait qu'elle comprend, dans un milieu approprié pour la teinture :

- au moins une première base d'oxydation choisie parmi la paraphénylènediamine, la 2-méthyl paraphénylènediamine et leurs sels d'addition avec un acide,

- au moins un coupleur choisi parmi les dérivés de métaphénylènediamine de formule (I) suivante :

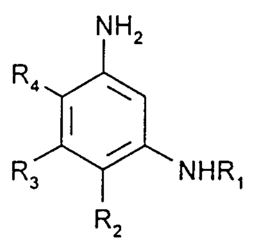

(I)

dans laquelle :

$R_1$ représente un atome d'hydrogène, un radical alkyle en $C_1$-$C_4$, monohydroxyalkyle en $C_1$-$C_4$ ou polyhydroxyalkyle en $C_2$-$C_4$ ;
$R_2$ et $R_3$, identiques ou différents, représentent un atome d'hydrogène, un radical alkyle en $C_1$-$C_4$ ou hydroxyalkoxy en $C_1$-$C_4$ ;
$R_4$ représente un radical alkoxy en $C_1$-$C_4$, aminoalkoxy en $C_1$-$C_4$, monohydroxyalkoxy en $C_1$-$C_4$, polyhydroxyalkoxy en $C_2$-$C_4$ ou un radical 2,4-diaminophénoxyalkoxy, sous réserve que lorsque $R_1$ désigne un atome d'hydrogène, alors $R_2$ et $R_4$ ne désignent pas simultanément un radical β-hydroxyéthyloxy, et que lorsque $R_1$, $R_2$ et $R_3$ désignent simultanément un atome d'hydrogène, alors $R_4$ est différent de méthoxy, et leurs sels d'addition acide,

- et au moins une deuxième base d'oxydation choisie parmi les dérivés de paraphénylènediamine tertiaire de formule (II) suivante :

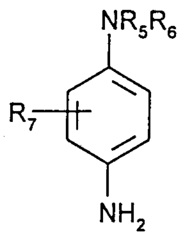

(II)

dans laquelle :

$R_5$ représente un radical alkyle en $C_1$-$C_4$, monohydroxyalkyle en $C_1$-$C_4$ ou polyhydroxyalkyle en $C_2$-$C_4$ ;
$R_6$ représente un radical monohydroxyalkyle en $C_1$-$C_4$ ou polyhydroxyalkyle en $C_2$-$C_4$ ;
$R_7$ représente un atome d'hydrogène, un radical alkyle en $C_1$-$C_4$, alkoxy en $C_1$-$C_4$ ou un atome d'halogène,
et leurs sels d'addition avec acide.

**2.** Composition selon la revendication 1, caractérisée par le fait que les sels d'addition avec un acide sont choisis parmi les chlorhydrates, les bromhydrates, les sulfates et les tartrates.

**3.** Composition selon la revendication 1 ou 2, caractérisée par le fait que les dérivés de métaphénylènediamine formule (I) sont choisis parmi le 3,5-diamino 1-éthyl 2-méthoxybenzène, le 3,5-diamino 2-méthoxy 1-méthyl benzène, le 2,4-diamino 1-éthoxybenzène, le 1,3-bis-(2,4-diaminophénoxy) propane, le bis-(2,4-diaminophénoxy) méthane, le 1-(β-aminoéthyloxy) 2,4-diamino benzène, le 2-amino 1-(β-hydroxyéthyloxy) 4-méthylamino benzène, le 2,4-diamino 1-éthoxy 5-méthyl benzène, le 2,4-diamino 5-(β-hydroxyéthyloxy) 1-méthylbenzène, le 2,4-diamino 1-(β,γ-dihydroxypropyloxy) benzène, le 2,4-diamino 1-(β-hydroxyéthyloxy) benzène, le 2-amino 4-N-(β-hydroxyéthyl) amino 1-méthoxy benzène, et leurs sels d'addition avec un acide.

**4.** Composition selon l'une quelconque des revendications 1 à 3, caractérisée par le fait que les dérivés de paraphénylènediamine tertiaire de formule (II) sont choisis parmi le 1-(N-éthyl, N'-β-hydroxyéthyl)amino 4-amino benzène, le 1-N,N'-bis-(β-hydroxyéthyl)amino 4-amino benzène, le 1-N,N'-bis-(β,γ-dihydroxypropyl)amino 4-amino benzène, et leurs sels d'addition avec un acide.

**5.** Composition selon l'une quelconque des revendications 1 à 4, caractérisée par le fait que la paraphénylènediamine et/ou la 2-méthyl paraphénylènediamine et/ou le ou les sels d'addition de ces composés avec un acide représentent de 0,0005 à 10 % en poids du poids total de la composition tinctoriale.

**6.** Composition selon la revendication 5, caractérisée par le fait que la paraphénylènediamine et/ou la 2-méthyl paraphénylènediamine et/ou le ou les sels d'addition de ces composés avec un acide représentent de 0,05 à 7 % en poids du poids total de la composition tinctoriale.

**7.** Composition selon l'une quelconque des revendications 1 à 6, caractérisée par le fait que le ou les dérivés de métaphénylénediamine formule (I) représentent de 0,0001 à 5 % en poids du poids total de la composition tinctoriale.

**8.** Composition selon la revendication 7, caractérisée par le fait que le ou les dérivés de métaphénylénediamine formule (I) représentent de 0,005 à 3 % en poids du poids total de la composition tinctoriale.

**9.** Composition selon l'une quelconque des revendications 1 à 8, caractérisée par le fait que le ou les dérivés de paraphénylènediamine tertiaire de formule (II) représentent de 0,0005 à 10 % en poids du poids total de la composition tinctoriale.

**10.** Composition selon la revendication 9, caractérisée par le fait que le ou les dérivés de paraphénylènediamine tertiaire de formule (II) représentent de 0,05 à 7 % en poids du poids total de la composition tinctoriale.

**11.** Composition selon l'une quelconque des revendications 1 à 10, caractérisée par le fait que ledit milieu approprié pour la teinture est constitué par de l'eau ou par un mélange d'eau et d'au moins un solvant organique choisi parmi les alcanols inférieurs en $C_1$-$C_4$, le glycérol, les glycols et éthers de glycols, les alcools aromatiques, les produits

analogues et leurs mélanges.

**12.** Composition selon l'une quelconque des revendications 1 à 11, caractérisée par le fait qu'elle présente un pH compris entre 2 et 12.

**13.** Composition selon l'une quelconque des revendications 1 à 12, caractérisée par le fait qu'elle contient, en outre, d'autres bases d'oxydation différentes de la paraphénylènediamine, de la 2-méthyl paraphénylènediamine, des dérivés de paraphénylènediamine tertiaire de formule (II) et de leurs sels d'addition avec un acide et/ou d'autres coupleurs différents des dérivés de métaphénylènediamines de formule (I) et de leurs sels d'addition avec un acide et/ou des colorants directs.

**14.** Procédé de teinture des fibres kératiniques et en particulier des fibres kératiniques humaines telles que les cheveux caractérisé par le fait que l'on applique sur ces fibres une composition tinctoriale telle que définie à l'une quelconque des revendications 1 à 13 et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

**15.** Procédé selon la revendication 14, caractérisé par le fait que l'agent oxydant présent dans la composition oxydante est choisi parmi le peroxyde d'hydrogène, le peroxyde d'urée, les bromates de métaux alcalins, les persels tels que les perborates et persulfates.

**16.** Dispositif à plusieurs compartiments ou "kit" de teinture à plusieurs compartiments dont un premier compartiment renferme une composition tinctoriale telle que définie à l'une quelconque des revendications 1 à 13 et un second compartiment renferme une composition oxydante comprenant un agent oxydant tel que défini à la revendication 15.

## Claims

**1.** Composition for the oxidation dyeing of keratinous fibres, and especially of human keratinous fibres such as hair, characterized in that it comprises, in a medium appropriate for dyeing:

- at least one first oxidation base chosen from para-phenylenediamine, 2-methyl-para-phenylenediamine and their addition salts with an acid,
- at least one coupler chosen from meta-phenylenediamine derivatives of formula (I) below:

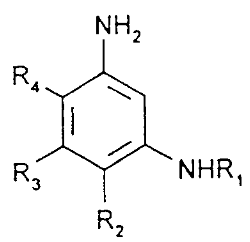

(I)

in which:

$R_1$ represents a hydrogen atom or a $C_1$-$C_4$ alkyl, $C_1$-$C_4$ monohydroxyalkyl or $C_2$-$C_4$ polyhydroxyalkyl radical;
$R_2$ and $R_3$, which are identical or different, represent a hydrogen atom or a $C_1$-$C_4$ alkyl or $C_1$-$C_4$ hydroxyalkoxy radical;
$R_4$ represents a $C_1$-$C_4$ alkoxy, $C_1$-$C_4$ aminoalkoxy, $C_1$-$C_4$ monohydroxyalkoxy or $C_2$-$C_4$ polyhydroxyalkoxy radical or a 2,4-diaminophenoxyalkoxy radical, with the proviso that, when $R_1$ denotes a hydrogen atom, $R_2$ and $R_4$ do not simultaneously denote a β-hydroxyethyloxy radical, and that, when $R_1$, $R_2$ and $R_3$ simultaneously denote a hydrogen atom,
$R_4$ is other than methoxy,
and their acid addition salts,

- and at least one second oxidation base chosen from tertiary para-phenylenediamine derivatives of formula (II)

below:

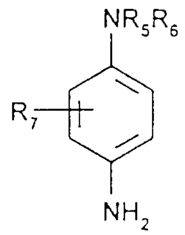

(II)

in which:

$R_5$ represents a $C_1$-$C_4$ alkyl, $C_1$-$C_4$ monohydroxyalkyl or $C_2$-$C_4$ polyhydroxyalkyl radical;
$R_6$ represents a $C_1$-$C_4$ monohydroxyalkyl or $C_2$-$C_4$ polyhydroxyalkyl radical;
$R_7$ represents a hydrogen atom, a $C_1$-$C_4$ alkyl or $C_1$-$C_4$ alkoxy radical or a halogen atom, and their addition salts with acid.

**2.** Composition according to Claim 1, characterized in that the addition salts with an acid are chosen from hydrochlorides, hydrobromides, sulphates and tartrates.

**3.** Composition according to Claim 1 or 2, characterized in that the meta-phenylenediamine derivatives of formula (I) are chosen from 3,5-diamino-1-ethyl-2-methoxybenzene, 3,5-diamino-2-methoxy-1-methylbenzene, 2,4-diamino-1-ethoxybenzene, 1,3-bis(2,4-diaminophenoxy)propane, bis(2,4-diamino- phenoxy)methane, 1-(β-aminoethyloxy)-2,4-diamino- benzene, 2-amino-1-(β-hydroxyethyloxy)-4-methylaminobenzene, 2,4-diamino-1-ethoxy-5-methylbenzene, 2,4 diamino-5-(β-hydroxyethyloxy)-1-methylbenzene, 2,4-diamino-1-(β,γ-dihydroxypropyl- oxy)benzene, 2,4-diamino-1-(β-hydroxyethyloxy)benzene, 2-amino-4-N-(β-hydroxyethyl)amino-1-methoxybenzene and their addition salts with an acid.

**4.** Composition according to any one of Claims 1 to 3, characterized in that the tertiary paraphenylenediamine derivatives of formula (II) are chosen from 1-(N-ethyl-N'-β-hydroxyethyl)amino-4-aminobenzene, 1-N,N'-bis(β-hydroxyethyl)amino-4-aminobenzene, 1-N,N'-bis(β,γ-dihydroxypropyl)amino-4-aminobenzene and their addition salts with an acid.

**5.** Composition according to any one of Claims 1 to 4, characterized in that the para-phenylenediamine and/or the 2-methyl-para-phenylenediamine and/or the addition salt or salts of these compounds with an acid represent from 0.0005 to 10 % by weight of the total weight of the dyeing composition.

**6.** Composition according to Claim 5, characterized in that the para-phenylenediamine and/or the 2-methyl-para-phenylenediamine and/or the addition salt or salts of these compounds with an acid represent from 0.05 to 7 % by weight of the total weight of the dyeing composition.

**7.** Composition according to any one of Claims 1 to 6, characterized in that the meta-phenylenediamine derivative or derivatives of formula (I) represent from 0.0001 to 5 % by weight of the total weight of the dyeing composition.

**8.** Composition according to Claim 7, characterized in that the meta-phenylenediamine derivative or derivatives of formula (I) represent from 0.005 to 3 % by weight of the total weight of the dyeing composition.

**9.** Composition according to any one of Claims 1 to 8, characterized in that the tertiary para-phenylenediamine derivative or derivatives of formula (II) represent from 0.0005 to 10 % by weight of the total weight of the dyeing composition.

**10.** Composition according to Claim 9, characterized in that the tertiary para-phenylenediamine derivative or derivatives of formula (II) represent from 0.05 to 7 % by weight of the total weight of the dyeing composition.

**11.** Composition according to any one of Claims 1 to 10, characterized in that the said medium appropriate for dyeing consists of water or of a mixture of water and at least one organic solvent chosen from $C_1$-$C_4$ lower alkanols, glyc-

erol, glycols and glycol ethers, aromatic alcohols, similar products and mixtures thereof.

**12.** Composition according to any one of Claims 1 to 11, characterized in that it has a pH of between 2 and 12.

**13.** Composition according to any one of Claims 1 to 12, characterized in that it additionally comprises oxidation bases other than para-phenylenediamine, 2-methyl-para-phenylenediamine, tertiary para-phenylenediamine derivatives of formula (II) and their addition salts with an acid and/or couplers other than meta-phenylenediamine derivatives of formula (I) and their addition salts with an acid and/or direct dyes.

**14.** Process for dyeing keratinous fibres, and especially human keratinous fibres such as hair, characterized in that a dyeing composition as defined in any one of Claims 1 to 13 is applied to these fibres and in that the colour is revealed at an acid, neutral or alkaline pH with the aid of an oxidizing agent which is added at the time of use to the dyeing composition or which is present in an oxidizing composition which is applied separately at the same time or sequentially.

**15.** Process according to Claim 14, characterized in that the oxidizing agent which is present in the oxidizing composition is chosen from hydrogen peroxide, urea peroxide, alkali metal bromates, and per salts such as perborates and persulphates.

**16.** Multi-compartment device or kit for dyeing, having a plurality of compartments, of which a first compartment contains a dyeing composition as defined in any one of Claims 1 to 13 and a second compartment contains an oxidizing composition comprising an oxidizing agent as defined in Claim 15.

**Patentansprüche**

**1.** Zusammensetzung zum oxidativen Färben von Keratinfasern und insbesondere von menschlichen Keratinfasern, wie dem Haar,
dadurch gekennzeichnet, daß
sie in einem zum Färben geeigneten Medium enthält:

- mindestens eine erste Oxidationsbase, die unter p-Phenylendiamin, 2-Methyl-p-Phenylendiamin und den Additionssalzen dieser Verbindungen mit einer Säure ausgewählt ist,

- mindestens einen Kuppler, der unter den m-Phenylendiaminderivaten der folgenden Formel (I) und den Additionssalzen dieser Verbindungen mit einer Säure ausgewählt ist,

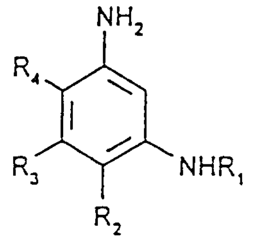

(I)

worin bedeuten:

$R_1$ Wasserstoff, $C_{1-4}$-Alkyl, $C_{1-4}$-Monohydroxyalkyl oder $C_{2-4}$-Polyhydroxyalkyl;
$R_2$ und $R_3$, die gleich oder voneinander verschieden sind, Wasserstoff, $C_{1-4}$-Alkyl oder $C_{1-4}$-Hydroxyalkoxy;
$R_4$ $C_{1-4}$-Alkoxy, $C_{1-4}$-Aminoalkoxy, $C_{1-4}$-Monohydroxyalkoxy, $C_{2-4}$-Polyhydroxyalkoxy oder eine Gruppe 2,4-Diaminophenoxyalkoxy,
mit der Maßgabe, daß $R_2$ und $R_4$ nicht gleichzeitig eine Gruppe β-Hydroxyethyloxy bedeuten, wenn $R_1$ ein Wasserstoffatom bedeutet, und daß $R_4$ von Methoxy verschieden ist, wenn $R_1$, $R_2$ und $R_3$ gleichzeitig Wasserstoff bedeuten, und

- mindestens eine zweite Oxidationsbase, die unter den tertiären p-Phenylendiaminderivaten der folgenden Formel (II) und den Additionssalzen dieser Verbindungen mit einer Säure ausgewählt ist:

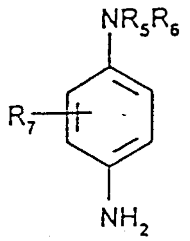

(II)

worin bedeuten:

$R_5$ $C_{1-4}$-Alkyl, $C_{1-4}$-Monohydroxyalkyl oder $C_{2-4}$-Polyhydroxyalkyl;
$R_6$ $C_{1-4}$-Monohydroxyalkyl oder $C_{2-4}$-Polyhydroxyalkyl;
$R_7$ Wasserstoff, $C_{1-4}$-Alkyl, $C_{1-4}$-Alkoxy oder ein Halogenatom.

**2.** Zusammensetzung nach Anspruch 1, dadurch gekennzeichnet, daß die Additionssalze mit einer Säure unter den Hydrochloriden, Hydrobromiden, Sulfaten und Tartraten ausgewählt sind.

**3.** Zusammensetzung nach Anspruch 1 oder 2, dadurch gekennzeichnet, daß die m-Phenylendiaminderivate der Formel (I) unter 3,5-Diamino-1-ethyl-2-methoxy-benzol, 3,5-Diamino-2-methoxy-1-methyl-benzol, 2,4-Diamino-1-ethoxy-benzol, 1,3-Bis(2,4-diaminophenoxy)-propan, Bis (2,4-diaminophenoxy)-methan, 1-(β-Aminoethyloxy)-2,4-diamino-benzol, 2-Amino-1-(β-hydroxyethyloxy)-4-methylamino-benzol, 2,4-Diamino-1-ethoxy-5-methylbenzol, 2,4-Diamino-5-(β-hydroxyethyloxy)-1-methylbenzol, 2,4-Diamino-1-(β,γ-dihydroxypropyloxy)-benzol, 2,4-Diamino-1-(β-hydroxyethyloxy)-benzol, 2-Amino-4-N-(β-hydroxyethyl)-amino-1-methoxy-benzol und den Additionssalzen dieser Verbindungen mit einer Säure ausgewählt sind.

**4.** Zusammensetzung nach einem der Ansprüche 1 bis 3, dadurch gekennzeichnet, daß die tertiären p-Phenylendiaminderivate der Formel (II) unter 1-(N-Ethyl, N-β-hydroxyethyl)amino-4-amino-benzol, 1-N,N-Bis(β-hydroxyethyl)amino-4-amino-benzol, 1-N,N-Bis(β,γ-dihydroxypropyl)amino-4-amino-benzol und den Additionssalzen dieser Verbindungen mit einer Säure ausgewählt sind.

**5.** Zusammensetzung nach einem der Ansprüche 1 bis 4, dadurch gekennzeichnet, daß das p-Phenylendiamin und/oder 2-Methyl-p-Phenylendiamin und/oder das oder die Additionssalze dieser Verbindungen mit einer Säure 0,0005 bis 10 Gew.-% des Gesamtgewichts der Färbemittelzusammensetzung ausmachen.

**6.** Zusammensetzung nach Anspruch 5, dadurch gekennzeichnet, daß das p-Phenylendiamin und/oder 2-Methyl-p-Phenylendiamin und/oder das oder die Additionssalze dieser Verbindungen mit einer Säure 0,05 bis 7 Gew.-% des Gesamtgewichts der Färbemittelzusammensetzung ausmachen.

**7.** Zusammensetzung nach einem der Ansprüche 1 bis 6, dadurch gekennzeichnet, daß das oder die m-Phenylendiaminderivate der Formel (I) 0,0001 bis 5 Gew.-% des Gesamtgewichts der Färbemittelzusammensetzung ausmachen.

**8.** Zusammensetzung nach Anspruch 7, dadurch gekennzeichnet, daß das oder die m-Phenylendiaminderivate der Formel (I) 0,005 bis 3 Gew.-% des Gesamtgewichts der Färbemittelzusammensetzung ausmachen.

**9.** Zusammensetzung nach einem der Ansprüche 1 bis 8, dadurch gekennzeichnet, daß das oder die tertiären p-Phenylendiaminderivate der Formel (II) 0,0005 bis 10 Gew.-% des Gesamtgewichts der Färbemittelzusammensetzung ausmachen.

**10.** Zusammensetzung nach Anspruch 9, dadurch gekennzeichnet, daß das oder die tertiären m-Phenylendiaminderivate der Formel (II) 0,05 bis 7 Gew.-% des Gesamtgewichts der Färbemittelzusammensetzung ausmachen.

**11.** Zusammensetzung nach einem der Ansprüche 1 bis 10, dadurch gekennzeichnet, daß das zum Färben geeignete Medium aus Wasser oder einem Gemisch aus Wasser und mindestens einem organischen Lösungsmittel besteht, das unter den niederen Alkanolen mit 1 bis 4 Kohlenstoffatomen, Glycerin, Glykolen und Glykolethern, aromatischen Alkoholen, analogen Produkten und deren Gemischen ausgewählt ist.

**12.** Zusammensetzung nach einem der Ansprüche 1 bis 11, dadurch gekennzeichnet, daß sie einen pH-Wert im Bereich von 2 bis 12 aufweist.

**13.** Zusammensetzung nach einem der Ansprüche 1 bis 12, dadurch gekennzeichnet, daß sie ferner weitere Oxidationsbasen, die von p-Phenylendiamin, 2-Methyl-p-Phenylendiamin, den tertiären p-Phenylendiaminderivaten der Formel (II) und den Additionssalzen dieser Verbindungen mit einer Säure verschieden sind, und/oder weitere Kuppler, die von den m-Phenylendiaminderivaten der Formel (I) und den Additionssalzen dieser Verbindungen mit einer Säure verschieden sind, und/oder Direktfarbstoffe enthält.

**14.** Verfahren zum Färben von Keratinfasern und insbesondere von menschlichen Keratinfasern, wie dem Haar, dadurch gekennzeichnet, daß auf die Fasern eine Färbemittelzusammensetzung nach einem der Ansprüche 1 bis 13 aufgetragen wird und die Farbe bei einem sauren, neutralen oder alkalischen pH-Wert mit Hilfe eines Oxidationsmittels entwickelt wird, das unmittelbar bei der Anwendung zu der Färbemittelzusammensetzung gegeben wird oder das in einer oxidierenden Zusammensetzung vorliegt, die gleichzeitig oder getrennt davon anschließend aufgebracht wird.

**15.** Verfahren nach Anspruch 14, dadurch gekennzeichnet, daß das in der oxidierenden Zusammensetzung vorliegende Oxidationsmittel unter Wasserstoffperoxid, Harnstoffperoxid, Alkalimetallbromaten und Salzen von Persäuren, wie Perboraten und Persulfaten, ausgewählt ist.

**16.** Vorrichtung mit mehreren Abteilungen oder "Kit" zum Färben mit mehreren Abteilungen, wobei eine erste Abteilung eine Färbemittelzusammensetzung nach einem der Ansprüche 1 bis 13 und eine zweite Abteilung eine oxidierende Zusammensetzung enthält, die das Oxidationsmittel, wie in Anspruch 15 definiert, enthält.